# EUROPÄISCHE PATENTSCHRIFT

(11) **EP 2 950 848 B1**
(45) Veröffentlichungstag und Bekanntmachung des Hinweises auf die Patenterteilung: **10.04.2019**
(21) Anmeldenummer: 14703312.0
(22) Anmeldetag: 31.01.2014
(51) Int. Cl.: A61M 1/36, A61M 39/28

(54) **VERBINDUNGSVORRICHTUNG MIT KLEMMVORRICHTUNG ZUM VERBINDEN MIT EINER ANORDNUNG ZUM VERSCHLIESSEN VON FLUSSWEGEN UND ZUSTANDSÜBERWACHUNG DER KLEMMVORRICHTUNG UND VERFAHREN HIERZU**
CONNECTING DEVICE WITH CLAMPING DEVICE FOR CONNECTION TO AN ARRANGEMENT FOR CLOSING OFF FLOW PATHS AND MONITORING THE STATE OF THE CLAMPING DEVICE AND METHOD THEREFOR
DISPOSITIF DE LIAISON COMPORTANT UN DISPOSITIF DE BLOCAGE À RELIER À UN DISPOSITIF DESTINÉ À FERMER DES VOIES FLUIDIQUES, DISPOSITIF DE CONTRÔLE DE L'ÉTAT DU DISPOSITIF DE BLOCAGE ET PROCÉDÉ CORRESPONDANT

(30) Priorität: 04.02.2013 DE 102013001850; 04.02.2013 US 201361760441 P
(43) Veröffentlichungstag der Anmeldung: 09.12.2015
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Weiß, Stefan
(86) Internationale Anmeldenummer: PCT/EP2014/051914
(87) Internationale Veröffentlichungsnummer: WO 2014/118324

(56) Entgegenhaltungen:
- DE-A1- 4 419 593
- DE-A1-102009 036 101
- DE-C1- 19 900 320
- US-A- 5 445 613
- US-A- 5 769 385
- US-A1- 2003 187 379
- US-A1- 2012 073 673
- US-B1- 6 604 908

## Beschreibung

Die vorliegende Erfindung betrifft eine Verbindungsvorrichtung mit einer externen Funktionseinrichtung zum Verbinden mit einer Anordnung und zum Verschließen von Flusswegen durch Aufnahme einer Klemmvorrichtung wobei eine Druckmesseinheit und/oder eine Längenmesseinheit zur Erfassung einer Klemmkraft und/oder einer Wegstrecke angepasst ist, so dass eine Zustandsüberwachung der Klemmvorrichtung ermöglicht wird.

Aus dem Stand der Technik sind Verbindungsvorrichtungen zum Verbinden einer externen Funktionseinrichtung mit einer Anordnung für Behandlungs-, Diagnoseverfahren und dergleichen, wie sie insbesondere im medizintechnischen Bereich eingesetzt werden, bekannt. Im Besonderen werden solche Verbindungsvorrichtungen in Anordnungen für die Behandlung von Blut in extrakorporalen Blutkreisläufen eingesetzt. Die Verbindungsvorrichtungen sind darüber hinaus mit diversen Komponenten ausgestattet, welche den Verbindungseinrichtungen eine definierte Funktionalität zuweisen. Bei diesen Komponenten handelt es sich zum Beispiel um Sensoren oder Aktoren, die eine ganz bestimmte Funktion innerhalb der Anordnung erfüllen (zum Beispiel Druckmessung). Des Weiteren beinhalten die bekannten Anordnungen noch Klemm- oder Ventilvorrichtungen zum Öffnen und Verschließen von Fluidwegen.

Für die Adaption von neuen, komplexeren Behandlungs-, Diagnoseverfahren oder dergleichen sind häufig weitere Klemm- oder Ventilvorrichtungen innerhalb einer Anordnung erforderlich.

Dabei ist es von Nachteil, dass sich zusätzliche Klemm- oder Ventilvorrichtungen nicht ohne weiteres in ein bestehendes Gerät integrieren lassen. Sofern also für bestimmte Verfahren alle geräteseitig vorhandenen Klemm- oder Ventilvorrichtungen belegt sind, aber zusätzlich noch Klemmfunktionen benötigt werden, so können diese Verfahren auf den vorhandenen Anordnungen nicht angewendet werden. Nachteilig ist weiterhin, dass die Anwendung solcher Verfahren dann nur durch eine umfangreiche konstruktive und technische Überarbeitung der Anordnung realisierbar ist. Dies ist mit einem erheblichen Kosten- und Zeitaufwand verbunden.

Aus der DE 10 2009 036 101 ist eine Verbindungsvorrichtung zum Verbinden wenigstens einer externen Funktionseinrichtung mit einer Anordnung mittels Verpressen der externen Funktionseinrichtung zwischen zwei Kontaktpunkten bekannt. Dabei ist die Verbindungseinrichtung mit einer Druckmesseinheit ausgestattet, zum Messen eines Drucks in einer Druckmessvorrichtung, welches die externe Funktionseinrichtung darstellt. Der Verbindungsvorrichtung ist, durch die Kombination der Druckmesseinheit mit der Druckmessvorrichtung, die definierte Funktion einer Druckmessung innerhalb der Anordnung zugeordnet.

Die DE 44 19 593 beschreibt eine Vorrichtung zum Messen eines Fluidrucks. Die beschriebene Vorrichtung ist mit einer Druckmesseinheit ausgestattet um einen Fluiddruck innerhalb einer Druckmessvorrichtung, zu messen. Auch hier ist der Verbindungseinrichtung in der Kombination mit der Druckmesseinheit und der Druckmessvorrichtung die definierte Funktion einer Druckmessung zugeordnet.

Die US 5,445,613 offenbart eine Klemmvorrichtung zum stufenweisen Verschließen eines flexiblen Schlauchs, der dabei zwischen zwei Klemmflächen verpresst wird.

Der Erfindung liegt nun die Aufgabe zugrunde, die aus dem Stand der Technik bekannten Verbindungsvorrichtungen zum Verbinden mit einer Anordnung derart zu konfigurieren, um Behandlungs-, Diagnoseverfahren und dergleichen, welchen komplexere Fluss-Schemata zugrunde liegen, durchführen zu können. Des Weiteren soll durch die Adaption der Verbindungsvorrichtungen eine flexible Anpassung der verschiedenen Verfahren an die vorhandene Anordnung und damit ein universeller Einsatz der Anordnung ermöglicht werden.

Darüber hinaus soll eine kostengünstige Adaption von komplexeren Behandlungs-, Diagnoseverfahren oder dergleichen zum Verbinden der Verbindungsvorrichtung und zum Verschließen von Flusswegen in einer bestehenden Anordnung zur Verfügung gestellt werden. Dabei soll die Anpassung an das jeweilige Verfahren sicher, schnell und einfach zu handhaben sein.

Es ist weiterhin die Aufgabe der Erfindung Komponenten der vorhandenen Anordnung derart anzupassen, um den Öffnungs- bzw. Schließzustand von Flusswegen überwachen zu können.

In Fällen, in denen für die Durchführung von Behandlungs-, Diagnoseverfahren oder dergleichen mehr als die vorhandenen Klemm- oder Ventileinrichtungen in einer Anordnung erforderlich sind, dagegen ein Teil der vorhandenen Verbindungsvorrichtungen, welchen ursprünglich eine andere Funktion zugeordnet ist (zum Beispiel der Druckmessung), nicht benötigt werden, besteht die Aufgabe darin, die Verbindungsvorrichtung, derart weiterzubilden, dass freie, nicht benötigte Verbindungsvorrichtungen innerhalb einer Anordnung eine andere, neue oder zusätzliche Funktion, anstelle ihrer originären, übernehmen können.

Die Aufgabe wird gelöst durch den Gegenstand des Anspruchs 1. Hierzu wird in eine Verbindungseinrichtung mit einer externen Funktionseinrichtung zum Verbinden mit einer Anordnung enthaltend eine Aufnahmeeinrichtung zur Aufnahme der externen Funktionseinrichtung, einem Außenträger, einem im Wesentlichen zylindrischen Innenträger und einem beweglichen Mittelträger, einer Druckmesseinheit zur Messung des Drucks in der externen Funktionseinrichtung und/oder einer Längenmesseinheit zum Messen einer Wegstrecke, einer Kraftübertragungseinrichtung zum kontinuierlichen Verschieben des beweglichen Mittelträgers entlang der Wegstrecke, eine externe Funktionseinrichtung eingelegt. Die externe Funktionseinrichtung ist in Form von Klemm- oder Ventilvorrichtungen ausgebildet und mit Klemmelementen versehen, zum Verschließen eines Flussweges.

Mittels diverser Komponenten, wie einer Druckmesseinheit zur Erfassung einer Klemm- oder Schließkraft und/oder einer Längenmesseinheit zur Messung einer Wegstrecke zwischen einem ersten Endpunkt und einem zweiten Endpunkt, können diese derart angepasst sein, um so eine Zustandsüberwachung der Klemmvorrichtung zu ermöglichen.

Durch den Einsatz einer externen Funktionseinrichtung, in Form von Klemm- oder Ventilvorrichtungen kann, eine kostengünstige Adaption von Behandlungs-, Diagnoseverfahren oder dergleichen in Verbindungseinrichtungen zum Verbinden der Verbindungsvorrichtung und zum Verschließen von Flusswegen in einer bestehenden Anordnung zur Verfügung gestellt werden.

In einer alternativen Ausführungsform kann neben der Klemmkraft- oder Schließkraft und/oder einer Wegstrecke auch ein Druck innerhalb eines fluidführenden Elements einer Klemm- oder Ventilvorrichtung bestimmt werden.

Die so konfigurierte Verbindungsvorrichtung wird anstelle ihrer ursprünglichen Funktion, mit einer anderen, neuen oder weiteren Funktionalität belegt. Die erfindungsgemäße Aufgabe wird weiterhin durch ein Verfahren nach den Ansprüchen 9 bis 11 gelöst.

Weitere vorteilhafte Ausführungsformen der Erfindung ergeben sich aus den Unteransprüchen.

Unter dem Begriff "komplexe Behandlungs-, Diagnoseverfahren oder dergleichen" sind solche Verfahren zu verstehen, die aufgrund ihrer verfahrensspezifischen Anforderungen mehr Klemm- bzw. Ventileinrichtungen benötigen als in den entsprechenden herkömmlichen Anordnungen vorhanden sind.

Bei den "externen Funktionseinrichtungen" wie sie im Sinne der Erfindung verwendet werden, handelt es sich um Klemm- oder Ventilvorrichtungen mit Klemm- bzw. Ventilelementen. Die Klemm- oder Ventilvorrichtungen können bevorzugt als Einmalartikel ausgeführt sein.

Unter "Klemm- oder Ventilvorrichtungen" versteht man Vorrichtungen, welche zum Absperren oder zur Regelung von Durchflüssen von Fluiden geeignet sind.

Mit dem Begriff "Verschließen" ist das Verringern oder eine komplette Unterbrechung eines Fluidflusses in einem fluidführenden Element, im Sinne der Erfindung zu verstehen.

Eine "Anordnung" wie sie hier verwendet wird kann eine medizintechnische Anordnung, wie zum Beispiel eine Blutbehandlungsvorrichtung, eine Dialysevorrichtung, insbesondere eine Apherese- oder Plasma-Apheresevorrichtung, eine Anordnung in der Labortechnik, in der Arznei- oder Lebensmittelherstellung oder dergleichen, sein.

Der Begriff "Verbinden" gemäß der Erfindung kann ein funktionelles und/oder mechanisches Verbinden der externen Funktionseinrichtung, in Form einer Klemm- oder Ventilvorrichtung mit einer Anordnung sein, wobei die Klemmvorrichtung mit mindestens einer Messeinrichtung auf der Seite der Anordnung in Kontakt steht um eine Klemm- oder Schließkraft und/oder eine Wegstrecke zu bestimmen.

Eine "Kraftübertragungseinrichtung", wie sie hier verwendet wird, dient zur Übertragung einer Kraft innerhalb einer Anordnung auf Komponenten in der Verbindungsvorrichtung. Die Kraft kann dabei auf mechanischem, pneumatischem, hydraulischem, elektrischem, elektromagnetischem, induktivem oder auf jedem anderen geeigneten Weg übertragen werden.

Bei den "Komponenten" zur Zustandsüberwachung, gemäß der Erfindung, handelt es sich vorrangig um Sensoren oder Aktoren, insbesondere um Messeinheiten, wie zum Beispiel Druckmesseinheiten und Längenmesseinheiten.

Die "Längenmesseinheit" wie sie im Sinne der Erfindung genutzt wird ist an den bewegten Teilen der Verbindungsvorrichtung angebracht. In einer besonderen Ausführungsform befindet sich diese im Außenträger der Verbindungsvorrichtung. In einem beweglichen Mittelträger der Verbindungsvorrichtung ist dazu eine Blende angebracht, über die mittels einer Lichtschranke eine Wegstrecke (S) erfasst werden kann um darüber verschiedene Öffnungs- und Schließzustände der Klemmvorrichtung bestimmen zu können.

Die "Druckmesseinheit" wie sie hier verwendet wird ist in erster Linie derart angepasst, dass diese als Gegenlager für die Klemmvorrichtung dient, um eine Klemm- bzw. Schließkraft zu erfassen. Die Druckmesseinheit kann in Kombination mit der Zustandsüberwachung der Klemmvorrichtung ebenfalls zur Messung eines Fluiddrucks eingesetzt werden.

Vorteilhafterweise kann durch Adaption der Klemmvorrichtung die Anordnung flexibel und universell für eine Vielzahl von Behandlungs-, Diagnoseverfahren oder dergleichen eingesetzt werden, welche mehr als die in der Anordnung vorhanden Klemmeinrichtungen benötigen. Die Anpassung der Verbindungsvorrichtung in einer Anordnung an die jeweiligen spezifischen Anforderungen für ein Verfahren, hinsichtlich der benötigten Klemmfunktionen, ist dabei schnell, einfach und sicher durchführbar. Darüber hinaus müssen keine aufwändigen und kostenintensiven bautechnischen Maßnahmen an einer Anordnung durchgeführt werden.

Im Folgenden werden die verschiedenen Ausführungsbeispiele der Erfindung unter Bezugnahme auf die Zeichnungen näher erläutert.
Figur 1 zeigt eine Schnittdarstellung einer Verbindungseinrichtung zum Verbinden einer externen Funktionseinrichtung mit einer Anordnung und eingelegter externer Funktionseinrichtung.
Figur 2 zeigt eine Schnittdarstellung einer Verbindungsvorrichtung mit einer Klemmvorrichtung als externer Funktionseinrichtung, welche beispielhaft ein Klemmelement in Form einer geöffneten Schlauchklemme und einem fluidführenden Element enthält.
Figuren 3a und 3b zeigen schematisch eine Klemmvorrichtung im geöffneten, durchströmten Zustand sowie im geschlossenen Zustand mit einer flexiblen unteren Seite sowie einer zusätzlich möglichen Druckmessung innerhalb der Klemmvorrichtung über die drucksensitive Membran der Druckmesseinheit.
Figur 4 zeigt beispielhaft ein Flussschema ohne adaptierte Verbindungsvorrichtungen.
Figur 5 zeigt beispielhaft ein Flussschema mit, durch Klemmvorrichtungen, adaptierten Verbindungsvorrichtungen.

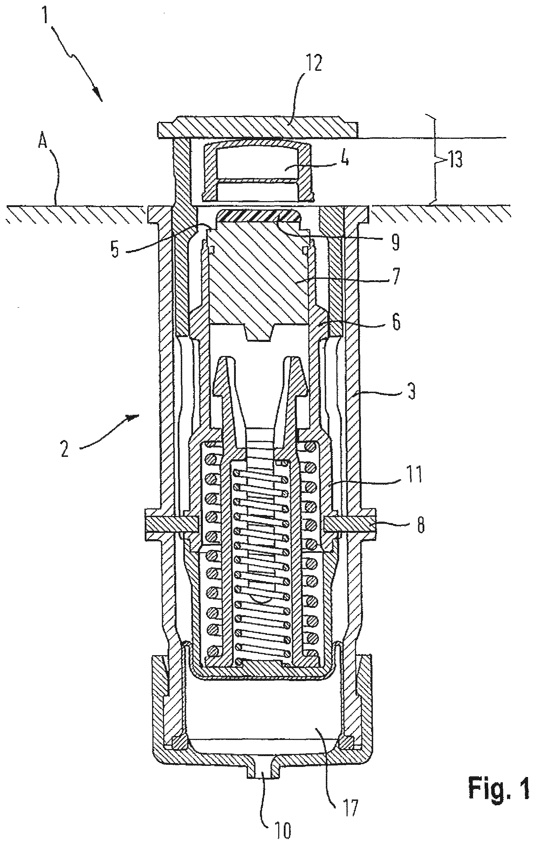

"Verbindungsvorrichtungen" (2) wie sie aus Figur 1 bekannt sind, enthalten drei ineinander angeordnete Hohlkörper. Der äußere der drei Hohlkörper wird als Außenträger (3) bezeichnet. Er kann stirnseitig starr, d.h. fest integriert, im Inneren einer Anordnung (1) beispielsweise einer Blutbehandlungsmaschine, auf Höhe einer Ankoppelfläche (A) zum Verbinden einer externen Funktionseinrichtung (4), angeflanscht sein.

Die Ankoppelfläche (A) ist ein Abschnitt auf der Oberseite eines Außenträgers (3). Sie kann allerdings auch eine äußere Sensorfläche oder ein Vorsprung (5) einer mit dem Innenträger (6) fest integrierten Druckmesseinheit (7) oder jeder zu einer Kraftaufnahme geeignete Abschnitt sein.

Im Inneren des Außenträgers (3) wird mittels starrer radialer Verbindungsträger (8) der im Wesentlichen zylindrische Innenträger (6) koaxial zum Außenträger (3) gehalten. Die vom Innenträger (6) getragene Druckmesseinheit (7) mit drucksensitiver Membran (9) ist gegenüber der Ankoppelfläche (A) stets in derselben Position angeordnet.

Zwischen dem Außenträger (3) und dem Innenträger (6) ist ein freier, gleichmäßiger ring- oder rohrförmiger Spalt vorgesehen. Das in Figur 1 dargestellte untere freie Ende des Außenträgers (3) ist durch einen Boden geschlossen und weist einen Kraftübertragungsanschluss (10) auf.

Als weitere, ineinander liegende Hohlkörper ist ein oberer und ein unterer Mittelträger (11) vorgesehen. Der untere Mittelträger ist in Fig. 1 als Federdübel dargestellt. Diese beiden Hohlkörper sind jeweils und getrennt voneinander axial innerhalb des Außenträgers (3) verschiebbar. Der Mittelträger (11) kann aber auch einstückig ausgebildet sein.

Im beweglichen Mittelträger (11) findet sich unterhalb einer Andruckplatte (12) eine Aussparung, welche als Aufnahmeeinrichtung (13) bezeichnet wird und zur Aufnahme der externen Funktionseinrichtung (4) vorgesehen ist.

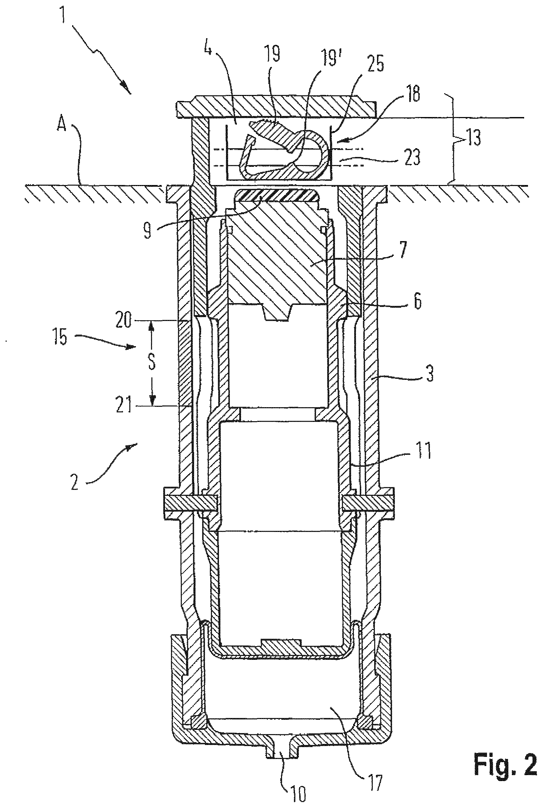

In Fig. 2 ist eine Verbindungseinrichtung (2) dargestellt, welche nun als externe Funktionseinrichtung (4) eine Klemm- oder Ventilvorrichtung (18), beispielhaft eine Schlauchklemme, im geöffneten Zustand zeigt, wobei die Klemmvorrichtung (18), ein fluidführendes Element (23) und Klemmelemente (19, 19') aufweist.

Zur Lösung der erfindungsgemäßen Aufgabe wird die Verbindungsvorrichtung (2) mit einer externen Funktionseinrichtung (4) zum Verbinden mit einer Anordnung (1) enthaltend
- eine Aufnahmeeinrichtung (13) zur Aufnahme der externen Funktionseinrichtung (4)
- einem Außenträger (3), einem im Wesentlichen zylindrischen Innenträger (6) und einem beweglichen Mittelträger (11)
- einer Druckmesseinheit (7) zur Messung des Drucks in der externen Funktionseinrichtung (4) und/oder
- einer Längenmesseinheit (15) zum Messen einer Wegstrecke (S)
- eine Kraftübertragungseinrichtung (17) zum kontinuierlichen Verschieben eines beweglichen Mittelträgers (11) entlang einer Wegstrecke (S), wobei die Verbindungsvorrichtung (2) derart konfiguriert ist, dass die externe Funktionseinrichtung (4) mit einer Klemmvorrichtung (18) zum Verschließen eines Flussweges ausgestattet ist, wobei
- die Klemmvorrichtung (18), welche auch als Ventilvorrichtung bezeichnet werden kann, über ein Gehäuse (25), ein fluidführendes Element (23) und Klemmelemente (19) verfügt. Als Klemmelemente können Druchflusskammern zum Einsatz kommen.

Weiterhin wird
- die Druckmesseinheit (7) zur Erfassung einer Klemm- oder Schließkraft und/oder
- die Längenmesseinheit (15) zur Messung einer Wegstrecke (S) zwischen einem ersten Endpunkt (20) und einem zweiten Endpunkt (21) derart angepasst ist um eine Zustandsüberwachung der Klemmvorrichtung (18) zu ermöglichen.

Zur Zustandsüberwachung wird eine Klemm- oder Schließkraft und/oder eine Wegstrecke (S) erfasst und mittels einer Auswerteeinheit (nicht dargestellt) der Öffnungs- bzw. Schließzustand der Klemmvorrichtungen (18) bestimmt. Neben der Überwachung, ob die Klemmvorrichtung (18) vollständig geöffnet oder geschlossen und damit der Flussweg frei durchgängig bzw. verschlossen ist, lassen sich ebenfalls Zwischenpositionen erfassen.

Mittels einer Kraftübertragungsvorrichtung (17) wird der Mittelträger (11) innerhalb des Außenträgers (3) der Verbindungsvorrichtung (2) verschoben, um so eine Kraft über die drucksensitive Membran (9) der Druckmesseinheit (7) und/oder eine Wegstrecke (S) über eine Längenmesseinheit (15) erfassen zu können. Die Kraftübertragung kann dabei insbesondere pneumatisch oder hydraulisch über einen Kraftübertragungsanschluss (10) erfolgen.

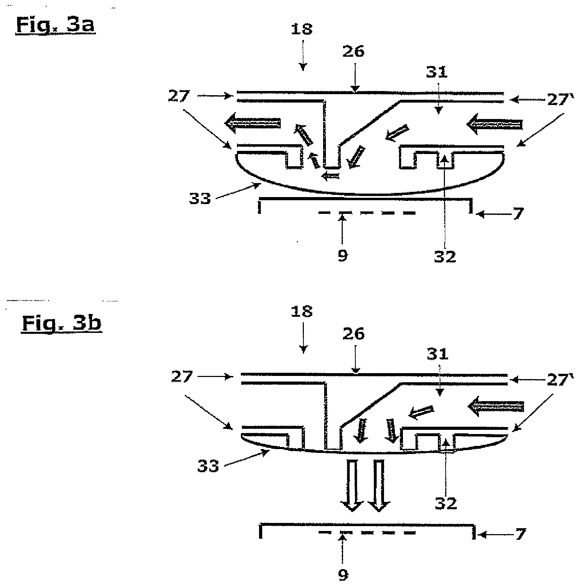

In einer erfindungsgemäßen Ausführungsform, wie in Fig. 3a schematisch dargestellt, kann die Klemmvorrichtung (18) als fluidführendes Element (23) eine Durchflusskammer (31) aufweisen, welche gleichzeitig auch das Gehäuse (25) der Klemmvorrichtung (18) darstellt. Die Durchflusskammer (31) enthält eine untere Seite (24), welche Dichtkonturen (32) aufweist. Die, den Dichtkonturen (32) zugewandte, untere Seite (24) kann durch eine flexible Membran (33) abgeschlossen werden. Die obere Seite (26), welche zur Andruckplatte (12) gerichtet ist, ist vorzugsweise durch eine starre Wand begrenzt. Die seitlich angeordneten Halterungen (27), (27') sind vorteilhafterweise starr ausgestaltet und bilden eine Strömungsverbindung, die vor und hinter der Durchflusskammer (31) in Form eines Schlauchs, Rohres, Kanals oder ähnlichem mit einer Anordnung (1) verbunden werden kann.

Im geöffneten Zustand liegt die Membran (33) nicht fluiddicht an der Unterseite der Dichtkonturen (32) an, so dass sich zwischen der Membran (33) und den Dichtkonturen (32) ein Zwischenraum ausbildet. Das in die Durchflusskammer (31) einströmende Fluid umströmt die Dichtkonturen (32) und ermöglicht dadurch einen Fluidfluss.

Die Dichtkonturen (32) können durch wenigstens zwei Ringe im Gehäuse gebildet werden, die durch einen Zwischenraum voneinander getrennt sind. Die Dichtkonturen (32) in der Durchflusskammer (31) der Klemmvorrichtung (18) sind vorzugsweise konzentrisch angeordnet, wobei diese kreisförmig oder von der Kreisform abweichend ausgebildet sein können und membranseitig in gleicher Höhe angeordnet sind.

Die Membran (33) kann an der unteren Seite (24), vorzugsweise am äußersten Ring der Dichtkontur (32) mit dem Gehäuse (25) der Klemmvorrichtung (18) verklebt, verschweißt, verklemmt oder verbördelt werden.

Eine Klemmwirkung wird, wie in Fig. 3b dargestellt, durch ein Andrücken der flexiblen Membran (33) an die Dichtkonturen (32) erreicht. Dabei kann, wie bereits oben dargelegt, mittels einer Kraftübertagungsvorrichtung (17) eine Aktivierungs- bzw. Stellkraft oder Gegenkraft Kraft auf den Mittelträger (11) der Verbindungsvorrichtung ausgeübt werden. Dies führt dazu, dass die flexible Membran (33) je nach der Stärke der Kraftaufwendung von der Aufnahmeeinrichtung (13) zwischen der Unterkante der Dichtkonturen (32) und der Druckmesseinheit (7) der Verbindungsvorrichtung (2) unterschiedlich stark verklemmt werden kann. Durch das Aufbringen einer Aktivierungs- bzw. Stellkraft wird die Andruckplatte (12) von der Ankoppelfläche (A) wegbewegt und die, in der Aufnahmeeinrichtung (13) eingelegte, Klemmvorrichtung (18) in einen geöffneten Zustand überführt. Beim Anlegen einer Gegenkraft bewegt sich die Andruckplatte (12) der Aufnahmeeinrichtung (13) dagegen auf die Ankoppelfläche (A) zu. Dabei wird die flexible Membran (33) der Klemmvorrichtung (18) gegen die Dichtkonturen (32), gedrückt, so dass sich der Spalt zwischen den Unterkanten der Dichtkonturen (32) und der flexiblen Membran (33) derart verkleinert, dass der Fluidfluss in der Durchflusskammer (31) verringert oder im vorzugsweise drucklosen Zustand, komplett unterbrochen werden kann.

Die, auf die flexible Membran (33) der Klemmvorrichtung (18), übertragene Klemm- oder Schließkraft wird über die drucksensitive Membran (9) der Druckmesseinheit (7) gemessen. Mittels einer Auswerteeinheit (nicht dargestellt) kann festgestellt werden, ob sich die Klemmvorrichtung (18) in einem geöffneten oder geschlossenen Zustand bzw. in einer Zwischenposition befindet.

Durch die erfindungsgemäß adaptierte Verbindungsvorrichtung (2) wird diese mittels der Bestimmung einer Klemm- oder Schließkraft durch die Druckmesseinheit (7) zur Zustandsüberwachung der Klemmvorrichtung (18) mit einer neuen Funktionalität als der ursprünglichen belegt, woraus die vorteilhaften Effekte der Erfindung resultieren.

Für den Fall, dass die Druckmesseinheit (7) die flexible Membran (33) fest an die Dichtkonturen (32) der Klemmvorrichtung (18) anpresst, ist ebenfalls eine Überwachung hinsichtlich eines geschlossenen Zustands der Klemmvorrichtung (18) über die Erfassung einer Wegstrecke (S) mittels einer Längenmesseinheit (15), wie bereits oben ausführlich beschrieben, möglich.

In einer weiteren besonderen alternativen Ausführungsform kann die Verbindungsvorrichtung (2) derart ausgebildet sein, dass neben einer Überwachung hinsichtlich eines geschlossenen Zustands der Klemmvorrichtung (18) über die Erfassung einer Wegstrecke (S) mittels einer Längenmesseinheit (15) auch eine Druckmessung in der Durchflusskammer (31) der Klemmvorrichtung (18) durchgeführt werden kann. Dabei kann der flexible Bodenbereich (30) der Klemmeinrichtung (18) mit der drucksensitiven Membran (9) der Druckmesseinheit (7) derart in Kontakt treten, dass im geschlossenen Zustand, d.h. wenn der Fluidfluss in der Durchflusskammer (31) unterbrochen ist, ein Fluiddruck in der Durchflusskammer (31) gemessen werden kann.

Auf Basis der, durch die Druckmessung, ermittelten Daten lassen sich Rückschlüsse auf die Funktionsweise weiterer Bauteile der Anordnung (1) ziehen, welche vor der Klemmvorrichtung (18) der adaptierten Verbindungsvorrichtung (2) angeordnet sind. So lässt sich durch die Druckmessung beispielsweise überprüfen, ob eine vorgeschaltete Pumpe ordnungsgemäß arbeitet bzw. das Vorratsbehältnis geöffnet ist oder ob möglicherwiese ein Strömungswiderstand oder eine Leckage in den fluidzuführenden Leitungen vorliegt.

Durch die erfindungsgemäß angepasste alternative Ausführungsform der Verbindungsvorrichtung (2) wird diese, neben der Zustandsüberwachung der Klemmvorrichtung (18) durch die Erfassung einer Wegstrecke (S) mittels einer Längenmesseinheit (15), als neuer Funktionalität, um die zusätzliche Funktion der Druckmessung erweitert.

In einer weiteren alternativen Ausführungsform können die Klemmvorrichtungen (18) als Einmalartikel ausgebildet sein.

Im Folgenden wird beispielhaft an einem Flussschema für eine extrakorporale Blutbehandlung, insbesondere einer Plasma-Apheresebehandlung, die Adaption der vorhandenen Verbindungseinrichtungen (2) mit Klemmvorrichtungen (18) zum Verbinden mit einer Anordnung (1) und zum Verschließen von Flusswegen sowie zur Zustandsüberwachung näher erläutert.

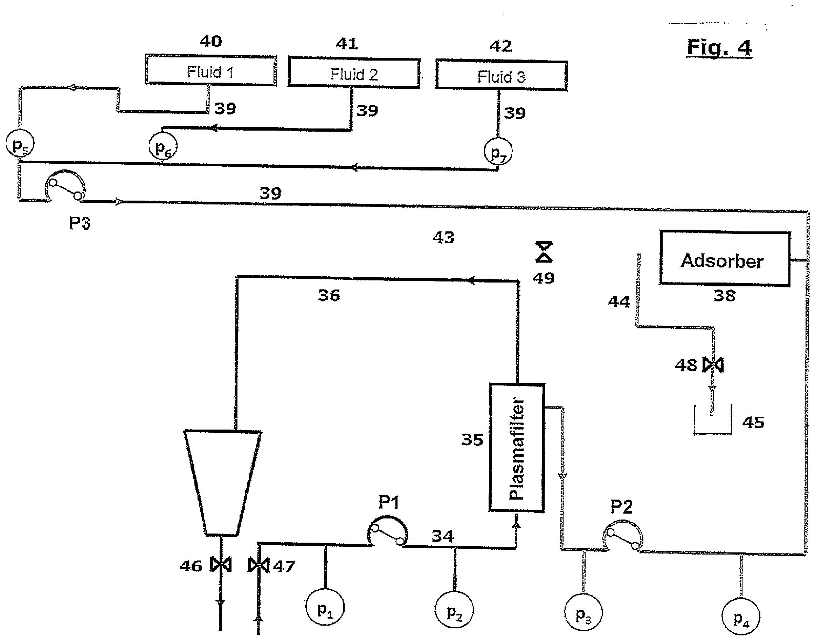

Bei Plasma-Aphereseverfahren, wie in Fig. 4 gezeigt, wird üblicherweise das Patientenblut mittels einer Pumpe (P1) über eine erste Blutleitung (34) in einen Plasmafilter (35) geleitet und das Plasma vom Blut abgetrennt. Die übrigen Blutbestandteile werden dem Patienten über eine zweite Blutleitung (36) wieder zugeführt. Das abgetrennte Plasma gelangt über eine Plasmaleitung (37) in einen Adsorber (38), wo unerwünschte Bestandteile gebunden werden und das so gereinigte Plasma über eine zweite Plasma- (43) und eine zweite Blutleitung (36) dem Patienten wieder zugeführt wird.

Um einen optimalen Behandlungserfolg gewährleisten zu können, ist es wichtig, die Flussrate in den einzelnen Behandlungsabschnitten durch Druckmessung exakt zu überwachen. In den bekannten Anordnungen (1) sind daher an den Position p1 bis p7 Verbindungseinrichtungen (2) mit einer externen Funktionseinrichtung (4) zur Druckmessung vorgesehen.

Bei komplexen Plasma-Aphereseverfahren welche zum Beispiel mit regenerierbaren Adsorbern (38) arbeiten, muss dieser bei einer gewissen Beladung gereinigt bzw. regeneriert werden. Die Regeneration des Adsorbers (38) kann u.U. während der Behandlung des Patienten erforderlich werden. Um dem Patienten, durch einen Umbau der Anordnung, ein zusätzliches Ab- und Ankoppeln zu ersparen, soll die Adsorberregeneration möglichst während der Behandlung durchführbar sein. Dazu müssen nacheinander verschiedene Lösungen über das Adsorbermaterial geleitet werden. Die benötigten Behältnisse (40, 41, 42) mit dem Regenerationsfluid können hierfür an die Fluidleitungen (39) angehängt und über diese die einzelnen Regenerationsfluide in den Adsorber (38) geleitet werden. In der Regel werden zunächst mit einer starken Säure die, aus dem Plasma, adsorbierten Bestandteile von dem Adsorbermaterial gewaschen. Im Anschluss wird das Adsobermaterial neutralisiert und abschließend mit einer physiologischen Lösung gespült. Das verbrauchte Regenerationsfluid wird über eine Drainageleitung (44) in ein Drainagebehältnis (45) abgelassen.

Da es sich bei den Reinigungslösungen teilweise um aggressive, nicht physiologische Lösungen handelt, ist es bei der Regeneration zwingend erforderlich, dass die Lösungen in einer bestimmten Reihenfolge in den Adsorber (38) geleitet werden und es während der Regenerationsphase zu keiner Durchmischung und/oder Einleitung der Reinigungslösungen in den Patienten kommt. Um während der Regeneration des Adsorbers (38) die Sicherheit des Patienten gewährleisten zu können, ist es daher zwingend notwendig, dass die Fluidleitungen (39) zu den Regenerationsfluidbehältnissen (40, 41, 42) sicher verschlossen werden können und eine Überwachung der Klemmfunktion möglich ist.

Die, in den bekannten Anordnungen (1), verwendeten Verbindungseinrichtungen (2), insbesondere die Verbindungseinrichtungen (2) an den Positionen p5 bis p7 in diesem Beispiel, sind mit Funktionseinrichtungen (4) zur Druckmessung ausgestattet und daher zum Verschließen von Fluidwegen und zur Zustandsüberwachung einer Klemmvorrichtung (18) ungeeignet.

Da in den bekannten Anordnungen (1) für den Regenerationsprozess keine zusätzlichen Klemmen zur Verfügung stehen, müssen die einzelnen Fluidleitungen (39) der Regenerationsfluide mittels manueller Klemmen verschlossen werden. Um diese zu bedienen, muss der Benutzer gezielt beim entsprechenden Verfahrensschritt alarmiert werden und eingreifen. Dies ist mit einer hohen Gefahr der Fehlbedienung verbunden.

Da während der Regenerationsphase die ursprünglich vorgesehenen Verbindungseinrichtungen (2) an den Positionen p5 bis p7 mit einer externen Funktionseinrichtung zur Druckmessung nicht, dafür aber zusätzliche Klemmen, benötigt werden, ist es mit den adaptierten Verbindungseinrichtungen (2) nun möglich, eine Funktionseinrichtung (4) mit Klemmvorrichtung (18) in eine Aufnahmeeinrichtung (13) einzusetzen und dabei eine Druckmesseinheit (7) zur Erfassung einer Klemm- oder Schließkraft und/oder eine Längenmesseinheit (15) zur Messung einer Wegstrecke (S) derart anzupassen um eine Zustandsüberwachung der Klemmvorrichtung (18) selbsttätig zu ermöglichen.

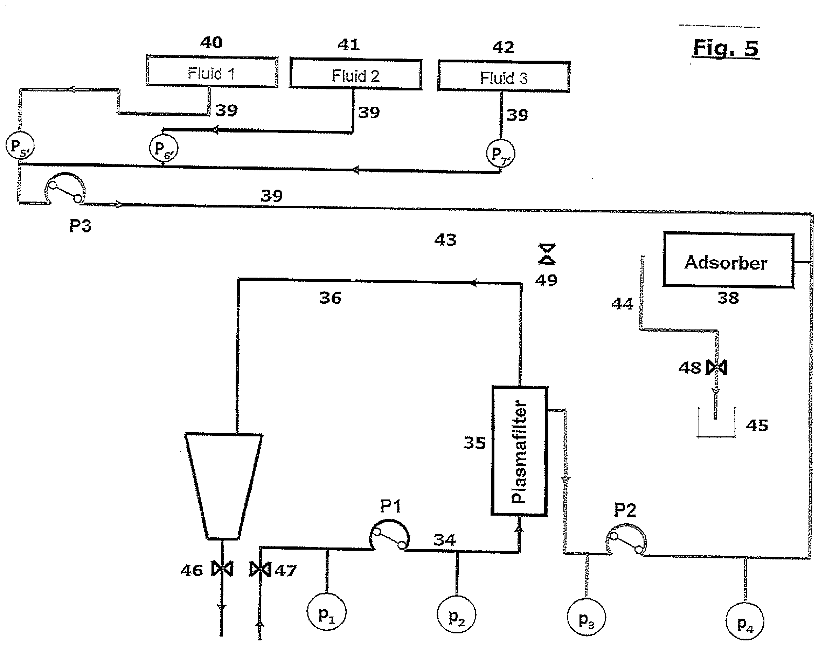

Fig. 5 zeigt das obige Flussschema mit den adaptierten Verbindungseinrichtungen (2) an den Positionen p5', p6' und p7' als externe Funktionseinrichtungen (4) zur Zustandsüberwachung der Klemmvorrichtungen (18).

Darüber hinaus können innerhalb einer Anordnung (1) alle Verbindungseinrichtungen (2), für die weitere Klemmvorrichtungen erforderlich sind, zum Beispiel für die Druckmesseinrichtungen an den Positionen p1, p2, p3 und/oder p4 wie beschrieben, angepasst werden. Dies kann auch der Fall sein, wenn in einem Behandlungs-, Diagnoseverfahren oder dergleichen zusätzliche Dialysat- oder Arzneimittellösungen verabreicht werden müssen.

### Bezugseichenliste

- 1: Anordnung
- 2: Verbindungsvorrichtung
- 3: Außenträger
- 4: Externe Funktionseinrichtung
- 5: Vorsprung
- 6: Innenträger
- 7: Druckmesseinheit
- 8: Verbindungsträger
- 9: drucksensitive Membran der Druckmesseinheit
- 10: Kraftübertragungsanschluss
- 11: Mittelträger
- 12: Andruckplatte
- 13: Aufnahmeeinrichtung
- 15: Längenmesseinheit
- 17: Kraftübertragungseinrichtung
- 18: Klemm-, Ventilvorrichtung
- 19, 19': Klemmelemente / Klemmprismen
- 20: erster Endpunkt
- 21: zweiter Endpunkt
- 23: fluidführendes Element

- 25: Gehäuse
- 26: obere Seite der Klemmvorrichtung
- 27, 27': seitliche Halterungen
- 31: Durchflusskammer
- 32: Dichtkonturen
- 33: flexible Membran
- 34: erste Blutleitung
- 35: Plasmafilter
- 36: zweite Blutleitung
- 37: erste Plasmaleitung
- 38: Adsorber
- 39: Fluidleitung
- 40, 41, 42: Regenerationsfluidbehältnisse
- 43: zweite Plasmaleitung
- 44: Drainageleitung
- 45: Drainagebehälter
- A: Ankoppelfläche
- B: Konstruktive Begrenzung
- P1, P2, P3: Pumpen
- p1-p7: Verbindungsvorrichtung mit Druckmessung
- p5', p6', p7': adaptierte Verbindungsvorrichtung mit Klemmvorrichtung
- S: Wegstrecke

## Patentansprüche

1. Verbindungseinrichtung (2) mit einer externen Funktionseinrichtung (4) zum Verbinden mit einer Anordnung (1), die Verbindungseinrichtung enthaltend
- eine Aufnahmeeinrichtung (13) zur Aufnahme der externen Funktionseinrichtung (4)
- einem Außenträger (3), einem im Wesentlichen zylindrischen Innenträger (6) und einem beweglichen Mittelträger (11)
- einer Druckmesseinheit (7) zur Messung des Drucks in der externen Funktionseinrichtung (4) und/oder
- einer Längenmesseinheit (15) zum Messen einer Wegstrecke (S)
- einer Kraftübertragungseinrichtung (17) zum kontinuierlichen Verschieben des beweglichen Mittelträgers (11) entlang der Wegstrecke (S), wobei
- die externe Funktionseinrichtung (4) eine Klemmvorrichtung (18) zum Verschließen eines Flussweges ist,
- die Klemmvorrichtung (18) über ein Gehäuse (25), ein fluidführendes Element (23) und Klemmelemente (19) verfügt, und
- die Druckmesseinheit (7) zur Erfassung einer Klemmkraft und/oder
- die Längenmesseinheit (15) zur Messung einer Wegstrecke (S) zwischen einem ersten Endpunkt (20) und einem zweiten Endpunkt (21) angepasst ist
- und so eine Zustandsüberwachung der Klemmvorrichtung (18) ermöglicht, **dadurch gekennzeichnet, dass**
- das fluidführende Element (23) eine Durchflusskammer (31) ist,
- die Klemmelemente (19) durch Dichtkonturen (32) im Gehäuse (25) gebildet werden,
- das Gehäuse (25) eine den Dichtkonturen (32) zugewandte, untere Seite (24) aufweist, welche durch eine flexible Membran (33) und eine obere Seite (26), welche durch eine starre Wand ausgebildet ist, begrenzt wird.

2. Verbindungsvorrichtung (2) nach Anspruch 1, **dadurch gekennzeichnet, dass** die Wegstrecke (S) zwischen einem ersten Endpunkt (20) und einem zweiten Endpunkt (21) durch die Entfernung zwischen einer starren Wand (26) der Klemmvorrichtung (18) im geöffneten Zustand und den Dichtkonturen (32) der Klemmvorrichtung (18) im geschlossenen Zustand festgelegt ist, welche über eine Längenmesseinheit (15) erfasst wird.

3. Verbindungsvorrichtung (2) nach Anspruch 2, **dadurch gekennzeichnet, dass** die Wegstrecke (S) zwischen der Klemmvorrichtung (18) im geöffneten Zustand und der Klemmvorrichtung (18) im geschlossenen Zustand durch die Abmessungen der Klemmvorrichtung (18) und der Aufnahmevorrichtung (13) festgelegt ist.

4. Verbindungseinrichtung (2) nach Anspruch 1, **dadurch gekennzeichnet, dass** die untere Seite (24) der Klemmvorrichtung (18) einen starren Bodenbereich (29) und einen flexiblen Bodenbereich (30) aufweist.

5. Verbindungseinrichtung (2) nach Anspruch 4, **dadurch gekennzeichnet, dass** der flexible Bodenbereich (30) der Klemmeinrichtung (18) im geschlossenen Zustand angepasst ist um mittels der drucksensitiven Membran (9) in der Druckmesseinheit (7) einen Fluiddruck in der Durchflusskammer (31) zu messen.

6. Verbindungseinrichtung (2) nach Anspruch 1, **dadurch gekennzeichnet, dass** die Dichtkonturen (32) durch wenigstens zwei Ringe im Gehäuse (25) der Klemmvorrichtung (18) gebildet werden.

7. Verbindungseinrichtung (2) nach Anspruch 6, **dadurch gekennzeichnet, dass** die Dichtkonturen (32) im Gehäuse (25) der Klemmvorrichtung (18) konzentrisch angeordnet sind, wobei diese kreisförmig oder von der Kreisform abweichend ausgebildet sein können und membranseitig auf gleicher Höhe angeordnet sind.

8. Verbindungseinrichtung (2) nach den Ansprüchen 4 und 7, **dadurch gekennzeichnet, dass** der flexible innere Bereich (30) mit dem starren Außenbereich (29) durch Schweißung, Klebung, Klemmung oder Bördelung verbunden ist.

9. Verfahren zum Verbinden wenigstens einer externen Funktionseinrichtung (4) in einer Verbindungseinrichtung (2) nach einem oder mehreren der vorangegangenen Ansprüche mit einer Anordnung (1), **dadurch gekennzeichnet, dass** mittels wenigstens einer Kraftübertragungsvorrichtung (17) ein beweglicher Mittelträger (11) entlang einer Wegstrecke (S) zwischen einem ersten Endpunkt (20) und einem zweiten Endpunkt (21) verschoben wird, wobei
- die verfahrene Wegstrecke (S) von einer Längenmesseinheit (15) und/oder
- die Klemm- oder Schließkraft von einer Druckmesseinheit (7) erfasst wird und
- mittels einer Auswerteeinheit eine Zustandsüberwachung der Klemmvorrichtung (18) möglich ist.

10. Verfahren nach Anspruch 9, **dadurch gekennzeichnet, dass** von der Längenmesseinheit (15) eine Wegstrecke (s) erfasst und mittels der drucksensitiven Membran (9) der Druckmesseinheit (7) eine Klemmkraft über den flexiblen Bodenbereich (30) gemessen wird und mittels einer Auswerteeinheit eine Zustandsüberwachung der Klemmvorrichtung (18) möglich ist.

11. Verfahren nach Anspruch 9, **dadurch gekennzeichnet, dass** mittels der Druckmesseinheit (7) zusätzlich ein Fluiddruck in den fluidführenden Elementen (23), insbesondere der Durchflusskammer (31) im geschlossenen Zustand bestimmt der Klemmvorrichtung (18) werden kann.

## Claims

1. A connecting device (2) having an external function device (4) for connecting to an arrangement (1) containing the connecting device
- a receptacle device (13) for accommodating the external function device (4)
- an outer carrier (3), an essentially cylindrical inner carrier (6), and a movable middle carrier (11)
- a pressure-measuring unit (7) for measuring the pressure in the external function device (4) and/or
- a length-measuring unit (15) for measuring a distance (S),
- a force-transmitting device (17) for continuous displacement of the movable middle carrier (11) along the distance (S), wherein
- the external function device (4) is a clamping device (18) for closing a flow path,
- the clamping device (18) has a housing (25), a fluid-carrying element (23) and clamping elements (19), and
- the pressure-measuring unit (7) for detecting a clamping force, and/or
- the length-measuring unit (15) is adjusted for measuring the distance (S) between a first end point (20) and a second end point (21),
- and thus permits monitoring of the condition of the clamping device (18),
**characterized in that**
- the fluid-carrying element (23) is a flow-through chamber (31),
- the clamping elements (19) are formed by sealing contours (32) in the housing (25),
- the housing (25) has a lower side (24), which faces the sealing contours (32) and is delimited by a flexible membrane (33), and an upper side (26) formed by a rigid wall.

2. The connecting device (2) according to claim 1, **characterized in that** the distance (S) between the first end point (20) and the second end point (21) is defined by the distance between a rigid wall (26) of the clamping device (18) in the open condition and the sealing contours (32) of the clamping device (18) in the closed condition, said distance being detected by a length-measuring unit (15).

3. The connecting device (2) according to claim 2, **characterized in that** the distance (S) between the clamping device (18) in the open condition and the clamping device (18) in the closed condition is defined by the dimensions of the clamping device (18) and of the receptacle device (13).

4. The connecting device (2) according to claim 1, **characterized in that** the lower side (24) of the clamping device (18) has a rigid bottom area (29) and a flexible bottom area (30).

5. The connecting device (2) according to claim 5, **characterized in that** the flexible bottom area (30) of the clamping device (18) in the closed state is adjusted in order to measure the fluid pressure in the flow-through chamber (31) by means of the pressure-sensitive membrane (9) in the pressure-measuring unit (7) .

6. The connecting device (2) according to claim 1, **characterized in that** the sealing contours (32) are formed by at least two rings in the housing (25) of the clamping device (18).

7. The connecting device (2) according to claim 6, **characterized in that** the sealing contours (32) are arranged concentrically in the housing (25) of the clamping device (18), wherein they may be designed to be circular or to deviate from the circular shape and are arranged at the same height on the membrane side.

8. The connecting device (2) according to claims 4 and 7, **characterized in that** the flexible inner area (30) is connected to the rigid outer area (29) by welding, gluing, clamping or flanging.

9. A method for connecting at least one external function device (4) to an arrangement (1) in a connecting device (2) according to any one or more of the preceding claims, **characterized in that** a movable middle carrier (11) displaced along a distance (S) between the first end point (20) and the second end point (21) by means of at least one force-transmitting device (17), wherein
- the distance (S) traveled is detected by a length-measuring unit (15), and/or
- the clamping or closing force is detected by a pressure-measuring unit (7), and
- it is possible to monitor the condition of the clamping device (18) by means of an evaluation unit.

10. The method according to claim 9, **characterized in that** the distance (S) is detected by the length-measuring unit (15), and a clamping force is measured by means of the pressure-sensitive membrane (9) of the pressure-measuring unit (7), and the condition of the clamping device (18) can be monitored by an evaluation unit.

11. The method according to claim 9, **characterized in that** the fluid pressure in the fluid-carrying elements (23), in particular the flow-through chamber (31), can additionally be determined by means of the pressure-measuring unit (7) in the closed condition of the clamping device (18).

## Revendications

1. Dispositif de connexion (2) à un dispositif fonctionnel externe (4) pour la connexion à un système (1), le dispositif de connexion comportant
- un dispositif récepteur (13) destiné à recevoir le dispositif fonctionnel externe (4),
- un support extérieur (3), un support intérieur (6) sensiblement cylindrique et un support central mobile (11),
- une unité de mesure de pression (7) pour mesurer la pression dans le dispositif fonctionnel externe (4) et/ou
- une unité de mesure de longueur (15) pour la mesure d'une trajectoire (S),
- un dispositif de transmission de force (17) pour déplacer en continu le support central mobile (11) le long de la trajectoire (S),
- le dispositif fonctionnel externe (4) étant un dispositif de serrage (18) servant à fermer une voie d'écoulement,
- le dispositif de serrage (18) disposant d'un boîtier (25), d'un élément conducteur de fluide (23) et d'éléments de serrage (19), et
- l'unité de mesure de pression (7) étant apte à détecter une force de serrage et/ou
- l'unité de mesure de longueur (15) étant apte à mesurer une trajectoire (S) entre un premier point terminal (20) et un second point terminal (21)
- et permettant ainsi une surveillance d'état du dispositif de serrage (18),
**caractérisé en ce que**
- l'élément conducteur de fluide (23) est une chambre d'écoulement (31),
- les éléments de serrage (19) sont constitués par des contours d'étanchéité (32) dans le boîtier (25),
- le boîtier (25) présentant une face inférieure (24) tournée vers les contours d'étanchéité (32) et qui est limitée par une membrane flexible (33) et une face supérieure (26) qui est constituée par une paroi rigide.

2. Dispositif de connexion (2) selon la revendication 1, **caractérisé en ce que** la trajectoire (S) entre un premier point terminal (20) et un second point terminal (21) est définie par l'éloignement entre une paroi rigide (26) du dispositif de serrage (18) en état ouvert et les contours d'étanchéité (32) du dispositif de serrage (18) en état fermé, qui est détecté par une unité de mesure de longueur (15).

3. Dispositif de connexion (2) selon la revendication 2, **caractérisé en ce que** la trajectoire (S) entre le dispositif de serrage (18) en état ouvert et le dispositif de serrage (18) en état fermé est définie par les dimensions du dispositif de serrage (18) et du dispositif récepteur (13).

4. Dispositif de connexion (2) selon la revendication 1, **caractérisé en ce que** la face inférieure (24) du dispositif de serrage (18) présente une partie fond rigide (29) et une partie fond souple (30).

5. Dispositif de connexion (2) selon la revendication 4, **caractérisé en ce que** la partie fond souple (30) du dispositif de serrage (18) en état fermé est apte à mesurer une pression de fluide dans la chambre d'écoulement (31) au moyen de la membrane sensible à la pression (9) dans l'unité de mesure de pression (7) .

6. Dispositif de connexion (2) selon la revendication 1, **caractérisé en ce que** les contours d'étanchéité (32) sont formés par au moins deux anneaux dans le boîtier (25) du dispositif de serrage (18).

7. Dispositif de connexion (2) selon la revendication 6, **caractérisé en ce que** les contours d'étanchéité (32) dans le boîtier (25) du dispositif de serrage (18) sont disposés concentriquement, ceux-ci pouvant être réalisés en une forme de cercle ou une forme différente de la forme de cercle et étant disposés à la même hauteur au niveau de la membrane.

8. Dispositif de connexion (2) selon les revendications 4 et 7, **caractérisé en ce que** la partie intérieure souple (30) est connectée à la partie extérieure rigide (29) par soudage, collage, serrage ou sertissage.

9. Procédé de connexion (2) d'au moins un dispositif fonctionnel externe (4) dans un dispositif de connexion (2) selon une ou plusieurs des revendications précédentes avec un système (1), **caractérisé en ce que**, au moyen d'au moins un dispositif de transfert de force (17), un support central mobile (11) est déplacé le long d'une trajectoire (17) entre un premier point terminal (20) et un second point terminal (21),
- la trajectoire parcourue (S) étant détectée par une unité de mesure de longueur (15) et/ou
- la force de serrage ou de fermeture étant détectée par une unité de mesure de pression (7) et
- une surveillance d'état du dispositif de serrage (18) étant possible au moyen d'une unité d'exploitation.

10. Procédé selon la revendication 9, **caractérisé en ce qu'**une trajectoire (S) est détectée par l'unité de mesure de longueur (15) et qu'une force de serrage est détectée au moyen de la membrane sensible à la pression (9) de l'unité de mesure de pression (7) au-dessus de la partie fond souple (30) et qu'une surveillance d'état du dispositif de serrage (18) est possible au moyen d'une unité d'exploitation.

11. Procédé selon la revendication 9, **caractérisé en ce que**, en outre, une pression (7) de fluide déterminée dans les éléments conducteurs de fluide (23), en particulier dans la chambre d'écoulement (31), peut être mesurée en état fermé du dispositif de serrage (18) .
